# EUROPEAN PATENT APPLICATION

(11) **EP 1 386 970 A1**
(43) Date of publication of application: **04.02.2004**
(21) Application number: 03016730.8
(22) Date of filing: 22.07.2003
(51) Int. Cl.: C12P 17/12

(54) **Combined utilization of genes in the biosynthetic pathway of caffeine**

(30) Priority: 23.07.2002 JP 2002213655
(71) Applicant: Nara Institute of Science and Technology, Ikoma-shi, Nara 630-0101 (JP)
(72) Inventor: Uefuji, Hirotaka, Nara-shi, Nara 631-0076 (JP); Sano, Hiroshi, Ikoma-shi, Nara 630-0114 (JP); Koizumi, Nozomu, Ikoma-shi, Nara 630-0101 (JP); Shinmyo, Atsuhiko, Ikoma-gun, Nara 636-0151 (JP)
(74) Representative: TER MEER STEINMEISTER & PARTNER GbR

(57) **Abstract**

In the biosynthetic reaction system of caffeine, enzymes which catalyze these reactions respectively, and a method of composite utilization of genes encoding these enzymes, respectively are provided. A process for producing 7-methylxanthine, theobromine or caffeine which comprises methylation of xanthosine, ribose removal of 7-methylxanthosine, methylation of 7-methylxanthine, and/or methylation of theobromine *ex vivo*, in the presence of a combination of two or more of the enzymes a, c and d, and the cellular extract b. a: an enzyme which catalyzes methylation of xanthosine at the 7-position of the purine ring and has the amino acid sequence set out in SEQ ID NO: 1, b: a crude cellular extract obtained from *Escherichia coli* which catalyzes ribose removal of 7-methylxanthosine at the 9-position of the purine ring, c: an enzyme which catalyzes methylation of 7-methylxanthine at the 3-position of the purine ring and has the amino acid sequence set out in SEQ ID NO: 4, d: an enzyme which catalyzes methylation of theobromine at the 1-position of the purine ring and has the amino acid sequence set out in SEQ ID NO: 7.

SEQUENCE LISTING

<110> NARA INSTITUTE OF SCIENCE AND TECHNOLOGY

<120> COMPOSITE UTILIZATION OF A GROUP OF GENES IN BIOSYNTHETIC PATHWAY OF CAFFEINE

<130> 19-024

<140> EP 03 016 730.8
<141> 2003-07-22

<150> JP2002-213655
<151> 2002-07-23

<160> 9

<170> Microsoft Word

<210> 1
<211> 372
<212> PRT
<213> Coffea arabica

<300>
<301> Ogawa, M., Herai, Y., Koizumi, N., Kusano, T., and Sano, H.
<302> 7-Methylxanthine Methyltransferase of Coffee Plants.Gene
      Isolation and Enzymatic Properties.
<303> Journal of Biological Chemistry
<304> 276
<305> 11
<306> 8213-8218
<307> 2001-03-16
<308> BAB39215
<309> 2000-09-11

<400> 1
Met Glu Leu Gln Glu Val Leu Arg Met Asn Gly Gly Glu Gly
                5                   10
Asp Thr Ser Tyr Ala Lys Asn Ser Ala Tyr Asn Gln Leu Val
15                  20                  25
Leu Ala Lys Val Lys Pro Val Leu Glu Gln Cys Val Arg Glu
    30                  35                  40
Leu Leu Arg Ala Asn Leu Pro Asn Ile Asn Lys Cys Ile Lys
        45                  50                  55
Val Ala Asp Leu Gly Cys Ala Ser Gly Pro Asn Thr Leu Leu
            60                  65                  70
Thr Val Arg Asp Ile Val Gln Ser Ile Asp Lys Val Gly Gln
                75                  80

Glu Lys Lys Asn Glu Leu Glu Arg Pro Thr Ile Gln Ile Phe
85                  90                  95
Leu Asn Asp Leu Phe Pro Asn Asp Phe Asn Ser Val Phe Lys
    100                 105                 110
Leu Leu Pro Ser Phe Tyr Arg Lys Leu Glu Lys Glu Asn Gly
        115                 120                 125
Arg Lys Ile Gly Ser Cys Leu Ile Gly Ala Met Pro Gly Ser
            130                 135                 140
Phe Tyr Ser Arg Leu Phe Pro Glu Glu Ser Met His Phe Leu
                145                 150
His Ser Cys Tyr Cys Leu Gln Trp Leu Ser Gln Val Pro Ser
155                 160                 165
Gly Leu Val Thr Glu Leu Gly Ile Ser Thr Asn Lys Gly Ser
    170                 175                 180
Ile Tyr Ser Ser Lys Ala Ser Arg Leu Pro Val Gln Lys Ala
        185                 190                 195
Tyr Leu Asp Gln Phe Thr Lys Asp Phe Thr Thr Phe Leu Arg
            200                 205                 210
Ile His Ser Glu Glu Leu Phe Ser His Gly Arg Met Leu Leu
                215                 220
Thr Cys Ile Cys Lys Gly Val Glu Leu Asp Ala Arg Asn Ala
225                 230                 235
Ile Asp Leu Leu Glu Met Ala Ile Asn Asp Leu Val Val Glu
    240                 245                 250
Gly His Leu Glu Glu Glu Lys Leu Asp Ser Phe Asn Leu Pro
        255                 260                 265
Val Tyr Ile Pro Ser Ala Glu Glu Val Lys Cys Ile Val Glu
            270                 275                 280
Glu Glu Gly Ser Phe Glu Ile Leu Tyr Leu Glu Thr Phe Lys
                285                 290
Val Leu Tyr Asp Ala Gly Phe Ser Ile Asp Asp Glu His Ile
295                 300                 305
Lys Ala Glu Tyr Val Ala Ser Ser Val Arg Ala Val Tyr Glu
    310                 315                 320
Pro Ile Leu Ala Ser His Phe Gly Glu Ala Ile Ile Pro Asp
        325                 330                 335
Ile Phe His Arg Phe Ala Lys His Ala Ala Lys Val Leu Pro
            340                 345                 350
Leu Gly Lys Gly Phe Tyr Asn Asn Leu Ile Ile Ser Leu Ala
                355                 360
Lys Lys Pro Glu Lys Ser Asp Val
365                 370

<210> 2
<211> 1316
<212> DNA
<213> Coffea arabica

<220>
<221> CDS
<222> (45)...(1163)

<300>
<308> AB048793
<309> 2000-09-11

<400> 2
ctttggcagt cccaatttga tttatgtaca agtcctgcat atgaatggag 50

ctccaagaag tcctgcggat gaatggaggc gaaggcgata caagctacgc 100

caagaattca gcctacaatc aactggttct cgccaaggtg aaacctgtcc 150

ttgaacaatg cgtacgggaa ttgttgcggg ccaacttgcc caacatcaac 200

aagtgcatta aagttgcgga tttgggatgc gcttctggac caaacacact 250

tttaacagtt cgggacattg tccaaagtat tgacaaagtt ggccaggaaa 300

agaagaatga attagaacgt cccaccattc agatttttct gaatgatctt 350

ttcccaaatg atttcaattc ggttttcaag ttgctgccaa gcttctaccg 400

caaacttgag aaagaaaatg gacgcaaaat aggatcgtgc ctaatagggg 450

caatgcccgg ctctttctac agcagactct tccccgagga gtccatgcat 500

tttttacact cttgttactg tcttcaatgg ttatctcagg ttcctagcgg 550

tttggtgact gaattgggga tcagtacgaa caaagggagc atttactctt 600

ccaaagcaag tcgtctgccc gtccagaagg catatttgga tcaatttacg 650

aaagatttta ccacatttct aaggattcat tcggaagagt tgttttcaca 700

tggccgaatg ctccttactt gcatttgtaa aggagttgaa ttagacgccc 750

ggaatgccat agacttactt gagatggcaa taaacgactt ggttgttgag 800

ggacatctgg aggaagaaaa attggatagt ttcaatcttc cagtctatat 850

accttcagca gaagaagtaa agtgcatagt tgaggaggaa ggttcttttg 900

aaattttata cctggagact tttaaggtcc tttacgatgc tggcttctct 950

attgacgatg aacatattaa agcagagtat gttgcatctt ccgttagagc 1000

agtttacgaa cccatcctcg caagtcattt tggagaagct attatacctg 1050

acatattcca caggtttgcg aagcatgcag caaaggttct ccccttgggc 1100

aaaggcttct ataataatct tatcatttct ctcgccaaaa agccagagaa 1150

gtcagacgtg taaaagtttg tttttgtgtt ggggaaagga ataagtgccg 1200

ttgggggtct ttcgggtatt gtgcttttta tattatattg ttttgtatcc 1250

gtaataaaag tggtgtgtaa gaataagata tttgacatat attattttca 1300

aaaaaaaaaa aaaaaa 1316

<210> 3
<211> 1316
<212> RNA
<213> Coffea arabica

<220>
<221> CDS
<222> (45)…(1163)

<300>
<308> AB048793
<309> 2000-09-11

<400> 3
cuuuggcagu cccaauuuga uuuauguaca aguccugcau augaauggag 50

cuccaagaag uccugcggau gaauggaggc gaaggcgaua caagcuacgc 100

caagaauuca gccuacaauc aacugguucu cgccaaggug aaaccugucc 150

uugaacaaug cguacgggaa uuguugcggg ccaacuugcc caacaucaac 200

aagugcauua aaguugcgga uuugggaugc gcuucuggac caaacacacu 250

uuuaacaguu cgggacauug uccaaaguau ugacaaaguu ggccaggaaa 300

agaagaauga auuagaacgu cccaccauuc agauuuuucu gaaugaucuu 350

uucccaaaug auuucaauuc gguuuucaag uugcugccaa gcuucuaccg 400

caaacuugag aaagaaaaug gacgcaaaau aggaucgugc cuaauagggg 450

caaugcccgg cucuuucuac agcagacucu uccccgagga guccaugcau 500

uuuuuacacu cuuguuacug ucuucaaugg uuaucucagg uuccuagcgg 550

uuuggugacu gaauugggga ucaguacgaa caaagggagc auuuacucuu 600

ccaaagcaag ucgucugccc guccagaagg cauauuugga ucaauuuacg 650

aaagauuuua ccacauuucu aaggauucau ucggaagagu uguuuucaca 700

uggccgaaug cuccuuacuu gcauuuguaa aggaguugaa uuagacgccc 750

ggaaugccau agacuuacuu gagauggcaa uaaacgacuu gguuguugag 800

ggacaucugg aggaagaaaa auuggauagu uucaaucuuc cagucuauau 850

accuucagca gaagaaguaa agugcauagu ugaggaggaa gguucuuuug 900

aaauuuuaua ccuggagacu uuuaaggucc uuuacgaugc uggcuucucu 950

auugacgaug aacauauuaa agcagaguau guugcaucuu ccguuagagc 1000

aguuuacgaa cccauccucg caagucauuu uggagaagcu auuauaccug 1050

acauauucca cagguuugcg aagcaugcag caaagguucu ccccuugggc 1100

aaaggcuucu auaauaaucu uaucauuucu cucgccaaaa agccagagaa 1150

gucagacgug uaaaaguuug uuuuuguguu ggggaaagga auaagugccg 1200

uugggggucu uucggguauu gugcuuuuua uauuauauug uuuuguaucc 1250

guaauaaaag ugguguguaa gaauaagaua uuugacauau auuauuuuca 1300

aaaaaaaaaa aaaaaa 1316

<210> 4
<211> 384
<212> PRT
<213> Coffea arabica

<400> 4
Met Glu Leu Gln Glu Val Leu His Met Asn Glu Gly Glu Gly
                5                   10
Asp Thr Ser Tyr Ala Lys Asn Ala Ser Tyr Asn Leu Ala Leu
15                  20                  25
Ala Lys Val Lys Pro Phe Leu Glu Gln Cys Ile Arg Glu Leu
    30                  35                  40

Leu Arg Ala Asn Leu Pro Asn Ile Asn Lys Cys Ile Lys Val
        45                  50                  55
Ala Asp Leu Gly Cys Ala Ser Gly Pro Asn Thr Leu Leu Thr
            60                  65                  70
Val Arg Asp Ile Val Gln Ser Ile Asp Lys Val Gly Gln Glu
                75                  80
Glu Lys Asn Glu Leu Glu Arg Pro Thr Ile Gln Ile Phe Leu
85                  90                  95
Asn Asp Leu Phe Gln Asn Asp Phe Asn Ser Val Phe Lys Leu
    100                 105                 110
Leu Pro Ser Phe Tyr Arg Lys Leu Glu Lys Glu Asn Gly Arg
        115                 120                 125
Lys Ile Gly Ser Cys Leu Ile Ser Ala Met Pro Gly Ser Phe
            130                 135                 140
Tyr Gly Arg Leu Phe Pro Glu Glu Ser Met His Phe Leu His
                145                 150
Ser Cys Tyr Ser Val His Trp Leu Ser Gln Val Pro Ser Gly
155                 160                 165
Leu Val Ile Glu Leu Gly Ile Gly Ala Asn Lys Gly Ser Ile
    170                 175                 180
Tyr Ser Ser Lys Ala Ser Arg Pro Pro Val Gln Lys Ala Tyr
        185                 190                 195
Leu Asp Gln Phe Thr Lys Asp Phe Thr Thr Phe Leu Arg Ile
            200                 205                 210
His Ser Lys Glu Leu Phe Ser Arg Gly Arg Met Leu Leu Thr
                215                 220
Cys Ile Cys Lys Val Asp Glu Tyr Asp Glu Pro Asn Pro Leu
225                 230                 235
Asp Leu Leu Asp Met Ala Ile Asn Asp Leu Ile Val Glu Gly
    240                 245                 250
His Leu Glu Glu Glu Lys Leu Ala Ser Phe Asn Leu Pro Phe
        255                 260                 265
Phe Thr Pro Ser Ala Glu Glu Val Lys Cys Ile Val Glu Glu
            270                 275                 280
Glu Gly Ser Phe Glu Ile Leu Tyr Leu Glu Thr Phe Lys Ala
                285                 290
His Tyr Asp Ala Gly Phe Ser Ile Asp Asp Asp Tyr Pro Val
295                 300                 305
Arg Ser His Phe Gln Val Tyr Gly Asp Glu His Ile Lys Ala
    310                 315                 320
Glu Tyr Val Ala Ser Leu Ile Arg Ser Val Tyr Glu Pro Ile
        325                 330                 335
Leu Ala Ser His Phe Gly Glu Ala Ile Met Pro Asp Leu Phe
            340                 345                 350
His Arg Leu Ala Lys His Ala Ala Lys Val Leu His Leu Gly
                355                 360
Lys Gly Cys Tyr Asn Asn Leu Ile Ile Ser Leu Ala Lys Lys
365                 370                 375

Pro Glu Lys Ser Asp Val
    380

<210> 5
<211> 1155
<212> DNA
<213> Coffea arabica

<220>
<221> CDS
<222> (1) ···(1152)

<400> 5
atggagctcc aagaagtcct gcatatgaat gaaggtgaag gcgatacaag        50

ctacgccaag aatgcatcct acaatctggc tcttgccaag gtgaaacctt       100

tccttgaaca atgcatacga gaattgttgc gggccaactt gcccaacatc       150

aacaagtgca ttaaagttgc ggatttggga tgcgcttctg gaccaaacac       200

acttttaaca gtgcgggaca ttgtgcaaag tattgacaaa gttggccagg       250

aagagaagaa tgaattagaa cgtcccacca ttcagatttt tctgaatgat       300

cttttccaaa atgatttcaa ttcggttttc aagttgctgc caagcttcta       350

ccgcaaactc gagaaagaaa atggacgcaa gataggatcg tgcctaataa       400

gcgcaatgcc tggctctttc tacggcagac tcttccccga ggagtccatg       450

cattttttgc actcttgtta cagtgttcat tggttatctc aggttcccag       500

cggtttggtg attgaattgg ggattggtgc aaacaaaggg agtatttact       550

cttccaaagc aagtcgtccg cccgtccaga aggcatattt ggatcaattt       600

acgaaagatt ttaccacatt tctaaggatt cattcgaaag agttgttttc       650

acgtggccga atgctcctta cttgcatttg taaagtagat gaatacgacg       700

aaccgaatcc cctagactta cttgacatgg caataaacga cttgattgtt       750

gagggacatc tggaggaaga aaaattggct agtttcaatc ttccattctt       800

tacaccttca gcagaagaag taaagtgcat agttgaggag gaaggttctt       850

ttgaaatttt atacctggag acttttaagg cccattatga tgctggcttc       900

tctattgatg atgattaccc agtaagatcc catttccaag tatacggcga 950

tgaacatatt aaagcagagt atgtggcatc attaattaga tcagtttacg 1000

aacccatcct cgcaagtcat tttggagaag ctattatgcc tgacttattc 1050

cacaggcttg cgaagcatgc agcaaaggtt ctccacttgg gcaaaggctg 1100

ctataataat cttatcattt ctctcgccaa aaagccagag aagtcagacg 1150

tgtaa 1155

<210> 6
<211> 1155
<212> RNA
<213> Coffea arabica

<220>
<221> CDS
<222> (1) ···(1152)

<400> 6
auggagcucc aagaaguccu gcauaugaau gaaggugaag gcgauacaag 50

cuacgccaag aaugcauccu acaaucuggc ucuugccaag gugaaaccuu 100

uccuugaaca augcauacga gaauuguugc gggccaacuu gcccaacauc 150

aacaagugca uuaaaguugc ggauuuggga ugcgcuucug gaccaaacac 200

acuuuuaaca gugcgggaca uugugcaaag uauugacaaa guuggccagg 250

aagagaagaa ugaauuagaa cgucccacca uucagauuuu ucugaaugau 300

cuuuuccaaa augauuucaa uucgguuuuc aaguugcugc caagcuucua 350

ccgcaaacuc gagaaagaaa auggacgcaa gauaggaucg ugccuaauaa 400

gcgcaaugcc uggcucuuuc uacggcagac ucuuccccga ggaguccaug 450

cauuuuuugc acucuuguua caguguucau ugguuaucuc agguucccag 500

cgguuuggug auugaauugg ggauuggugc aaacaaaggg aguauuuacu 550

cuuccaaagc aagucguccg cccguccaga aggcauauuu ggaucaauuu 600

acgaaagauu uuaccacauu ucuaaggauu cauucgaaag aguuguuuuc 650

acguggccga augcuccuua cuugcauuug uaaaguagau gaauacgacg 700

aaccgaaucc ccuagacuua cuugacaugg caauaaacga cuugauuguu 750

gagggacauc uggaggaaga aaaauuggcu aguuucaauc uuccauucuu 800

uacaccuuca gcagaagaag uaaagugcau aguugaggag gaagguucuu 850

uugaaauuuu auaccuggag acuuuuaagg cccauuauga ugcuggcuuc 900

ucuauugaug augauuaccc aguaagaucc cauuuccaag uauacggcga 950

ugaacauauu aaagcagagu auguggcauc auuaauuaga ucaguuuacg 1000

aacccauccu cgcaagucau uuuggagaag cuauuaugcc ugacuuauuc 1050

cacaggcuug cgaagcaugc agcaaagguu cuccacuugg gcaaaggcug 1100

cuauaauaau cuuaucauuu cucucgccaa aaagccagag aagucagacg 1150

uguaa 1155

<210> 7
<211> 384
<212> PRT
<213> Coffea arabica

<400> 7
Met Glu Leu Gln Glu Val Leu His Met Asn Gly Gly Glu Gly
                5                   10
Asp Thr Ser Tyr Ala Lys Asn Ser Phe Tyr Asn Leu Phe Leu
15                  20                  25
Ile Arg Val Lys Pro Ile Leu Glu Gln Cys Ile Gln Glu Leu
    30                  35                  40
Leu Arg Ala Asn Leu Pro Asn Ile Asn Lys Cys Ile Lys Val
        45                  50                  55
Ala Asp Leu Gly Cys Ala Ser Gly Pro Asn Thr Leu Leu Thr
            60                  65                  70
Val Arg Asp Ile Val Gln Ser Ile Asp Lys Val Gly Gln Glu
                75                  80
Lys Lys Asn Glu Leu Glu Arg Pro Thr Ile Gln Ile Phe Leu
85                  90                  95
Asn Asp Leu Phe Gln Asn Asp Phe Asn Ser Val Phe Lys Ser
    100                 105                 110
Leu Pro Ser Phe Tyr Arg Lys Leu Glu Lys Glu Asn Gly Arg
        115                 120                 125

Lys Ile Gly Ser Cys Leu Ile Gly Ala Met Pro Gly Ser Phe
            130                 135                 140
Tyr Gly Arg Leu Phe Pro Glu Glu Ser Met His Phe Leu His
                145                 150
Ser Cys Tyr Cys Leu His Trp Leu Ser Gln Val Pro Ser Gly
155                 160                 165
Leu Val Thr Glu Leu Gly Ile Ser Ala Asn Lys Gly Cys Ile
    170                 175                 180
Tyr Ser Ser Lys Ala Ser Arg Pro Pro Ile Gln Lys Ala Tyr
        185                 190                 195
Leu Asp Gln Phe Thr Lys Asp Phe Thr Thr Phe Leu Arg Ile
            200                 205                 210
His Ser Glu Glu Leu Ile Ser Arg Gly Arg Met Leu Leu Thr
                215                 220
Trp Ile Cys Lys Glu Asp Glu Phe Glu Asn Pro Asn Ser Ile
225                 230                 235
Asp Leu Leu Glu Met Ser Ile Asn Asp Leu Val Ile Glu Gly
    240                 245                 250
His Leu Glu Glu Glu Lys Leu Asp Ser Phe Asn Val Pro Ile
        255                 260                 265
Tyr Ala Pro Ser Thr Glu Glu Val Lys Cys Ile Val Glu Glu
            270                 275                 280
Glu Gly Ser Phe Glu Ile Leu Tyr Leu Glu Thr Phe Lys Val
                285                 290
Pro Tyr Asp Ala Gly Phe Ser Ile Asp Asp Asp Tyr Gln Gly
295                 300                 305
Arg Ser His Ser Pro Val Ser Cys Asp Glu His Ala Arg Ala
    310                 315                 320
Ala His Val Ala Ser Val Val Arg Ser Ile Phe Glu Pro Ile
        325                 330                 335
Val Ala Ser His Phe Gly Glu Ala Ile Met Pro Asp Leu Ser
            340                 345                 350
His Arg Ile Ala Lys Asn Ala Ala Lys Val Leu Arg Ser Gly
                355                 360
Lys Gly Phe Tyr Asp Ser Leu Ile Ile Ser Leu Ala Lys Lys
365                 370                 375
Pro Glu Lys Ser Asp Val
    380

<210> 8
<211> 1155
<212> DNA
<213> Coffea arabica

<220>
<221> CDS
<222> (1) ···(1152)

<400> 8
atggagctcc aagaagtcct gcatatgaat ggaggcgaag gcgatacaag 50

ctacgccaag aactcattct acaatctgtt tctcatcagg gtgaaaccta 100

tccttgaaca atgcatacaa gaattgttgc gggccaactt gcccaacatc 150

aacaagtgca ttaaagttgc ggatttggga tgcgcttctg gaccaaacac 200

acttttaaca gttcgggaca ttgtacaaag tattgacaaa gttggccagg 250

aaaagaagaa tgaattagaa cgtcccacca ttcagatttt tctgaatgat 300

cttttccaaa atgatttcaa ttcggttttc aagtcgctgc caagcttcta 350

ccgcaaactt gagaaagaaa atggacgcaa aataggatca tgcctgatag 400

gcgcaatgcc tggctctttc tacggcagac tcttccccga ggagtccatg 450

cattttttac actcttgtta ctgtttgcat tggttatctc aggttcccag 500

cggtttggtg actgaattgg ggatcagtgc gaacaaaggg tgcatttact 550

cttccaaagc aagtcgtccg cccatccaga aggcatattt ggatcaattt 600

acgaaagatt ttaccacatt tcttaggatt cattcggaag agttgatttc 650

acgtggccga atgctcctta cttggatttg caaagaagat gaattcgaga 700

acccgaattc catagactta cttgagatgt caataaacga cttggttatt 750

gagggacatc tggaggaaga aaaattggac agtttcaatg ttccaatcta 800

tgcaccttca acagaagaag taaagtgcat agttgaggag gaaggttctt 850

ttgaaatttt atacctggag acttttaagg tcccttatga tgctggcttc 900

tctattgatg atgattacca aggaagatcc cattccccag tatcctgcga 950

tgaacatgct agagcagcgc atgtggcatc tgtcgttaga tcaattttcg 1000

aacccatcgt cgcaagtcat tttggagaag ctatcatgcc tgacttatcc 1050

cacaggattg cgaagaatgc agcaaaggtt cttcgctccg gcaaaggctt 1100

ctatgatagt cttatcattt ctctcgccaa aaagccagag aagtcagacg 1150

tgtaa 1155

<210> 9
<211> 1155
<212> RNA
<213> Coffea arabica

<220>
<221> CDS
<222> (1) ···(1152)

<400> 9
auggagcucc aagaaguccu gcauaugaau ggaggcgaag gcgauacaag 50

cuacgccaag aacucauucu acaaucuguu ucucaucagg gugaaaccua 100

uccuugaaca augcauacaa gaauuguugc gggccaacuu gcccaacauc 150

aacaagugca uuaaaguugc ggauuuggga ugcgcuucug gaccaaacac 200

acuuuuaaca guucgggaca uuguacaaag uauugacaaa guuggccagg 250

aaaagaagaa ugaauuagaa cgucccacca uucagauuuu ucugaaugau 300

cuuuuccaaa augauuucaa uucgguuuuc aagucgcugc caagcuucua 350

ccgcaaacuu gagaaagaaa auggacgcaa aauaggauca ugccugauag 400

gcgcaaugcc uggcucuuuc uacggcagac ucuuccccga ggaguccaug 450

cauuuuuuac acucuuguua cuguuugcau ugguuaucuc agguucccag 500

cgguuuggug acugaauugg ggaucagugc gaacaaaggg ugcauuuacu 550

cuuccaaagc aagucguccg cccauccaga aggcauauuu ggaucaauuu 600

acgaaagauu uuaccacauu ucuuaggauu cauucggaag aguugauuuc 650

acguggccga augcuccuua cuuggauuug caaagaagau gaauucgaga 700

acccgaauuc cauagacuua cuugagaugu caauaaacga cuugguuauu 750

gagggacauc uggaggaaga aaaauuggac aguuucaaug uuccaaucua 800

ugcaccuuca acagaagaag uaaagugcau aguugaggag gaagguucuu 850

uugaaauuuu auaccuggag acuuuuaagg ucccuuauga ugcuggcuuc 900

ucuauugaug augauuacca aggaagaucc cauuccccag uauccugcga 950

ugaacaugcu agagcagcgc auguggcauc ugucguuaga ucaauuuucg 1000

aacccaucgu cgcaagucau uuuggagaag cuaucaugcc ugacuuaucc 1050

cacaggauug cgaagaaugc agcaaagguu cuucgcuccg gcaaaggcuu 1100

cuaugauagu cuuaucauuu cucucgccaa aaagccagag aagucagacg 1150

uguaa 1155

## Description

### BACKGROUND OF THE INVENTION

### 1. Field of the Invention

The present invention relates to xanthosine methyltransferase, 7-methylxanthine methyltransferase (theobromine synthase) and 3,7-dimethylxanthine methyltransferase (caffeine synthase) which catalyze respective steps in a series of a reaction system in which caffeine is synthesized from xanthosine via 7-methylxanthosine, 7-methylxanthine and theobromine, and a method of composite utilization of multiple genes in the biosynthetic pathway of caffeine encoding these enzymes, i.e., a method in which two or more of the multiple enzymes described above are used in combination and a method in which two or more of the multiple genes in the biosynthetic pathway of caffeine described above are used in combination.

### 2. Description of the Related Art

Caffeine is a purine alkaloid which is biologically synthesized in plant species such as coffee (*Coffea*), tea (*Camellia sinensis*), cola (*Cola acuminate*) and mate (*Ilex paraguariensis*). Caffeine has been widely utilized as a medicine because it has a variety of pharmacological actions such as central nervous system stimulatory action S . Further, potential ability of this compound capable of being utilized as a pesticide is suggested because it has feeding-repellent effects and pesticidal effects against insects and the like.

Caffeine is currently produced through extracting from the aforementioned plants or through chemical synthesis. Accordingly, development of techniques has been desired in which microorganisms or plants which do not biologically synthesize caffeine in themselves are allowed to produce caffeine through utilizing a gene recombination technique for the purpose of supplying caffeine on a large scale at a lower cost. In addition, such techniques to allow production of caffeine in plants are also expected as a process for directly defending the plant from pest feeding by a herbivore.

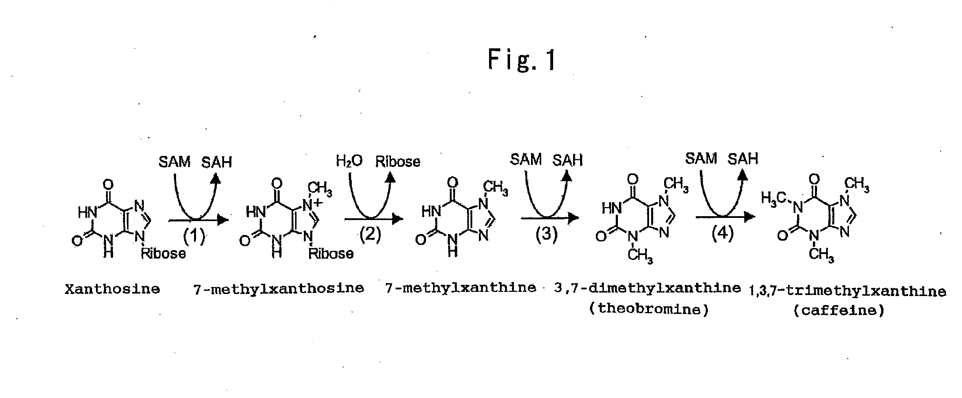

In coffee plants or tea plants, caffeine is biologically synthesized from xanthosine as a starting material, which is an intermediate catabolic product of adenine nucleotide and guanine nucleotide, via 7-methylxanthosine, 7-methylxanthine and theobromine (Fig. 1). A series of this reaction is catalyzed by xanthosine methyltransferase, an enzyme to remove the ribose moiety of 7-methylxanthosine, theobromine synthase and caffeine synthase. CaMXMT cDNA encoding the theobromine synthase was already isolated from coffee plants (see, Ogawa et al., J. Biol. Chem., 276, 8213-8218 (2001). Moreover, JP-A No. 2001-37490 describes the amino acid sequence of methyltransferase which catalyzes two steps of methylation reactions: 7-methylxanthine → theobromine → caffeine, and the nucleotide sequence encoding the same. Further, this gazette describes a process for modifying the constitution of methylxanthines in a plant which biologically synthesizes the methylxanthines (7-methylxanthine, paraxanthine, theobromine and caffeine) through introducing a DNA having the nucleotide sequence. However, any technique has not been known heretofore to allow this compound to be produced in a living organism species which can not biologically synthesize methylxanthines in itself.

### SUMMARY OF THE INVENTION

An object of the present invention is to provide enzymes respectively catalyze the reactions in a series of the reaction to produce caffeine from xanthosine via 7-methylxanthosine, 7-methylxanthine and theobromine, and a method of composite utilization of genes encoding these enzymes respectively. For example, following objects can be accomplished by the present invention. (1) To produce methylxanthines (7-methylxanthine, theobromine or caffeine) from xanthosine using two or more of the multiple enzymes in combination which catalyze each step in the series of the aforementioned caffeine biosynthetic reaction system. (2) To alter the metabolism of an organism which does not biologically synthesize methylxanthines in itself to allow for production of these compounds. (3) To defend a plant from pest feeding by a herbivore by the process (2) described above.

The present inventors elaborately investigated to solve the aforementioned problems, and obtained the following findings. First, DNA fragments having each of the nucleotide sequence set out in SEQ ID NO: 2, 5 and 8 in Sequence Listing were amplified by PCR. Next, these DNA fragments were incorporated into an expression vector, and the resulting recombinant vector was introduced into *Escherichia coli* to express a large amount of the recombinant protein derived from the DNA. After crude extraction or purification of this recombinant protein, enzymological characteristics thereof were studied. Accordingly, it was found that the protein derived from the nucleotide sequence set out in SEQ ID NO: 2 catalyzed a productive reaction of 7-methylxanthosine through methylation of xanthosine; that the protein derived from the nucleotide sequence set out in SEQ ID NO: 5 catalyzed a productive reaction of theobromine through methylation of 7-methylxanthine; and that the protein derived from the nucleotide sequence set out in SEQ ID NO: 8 catalyzed a productive reaction of caffeine through methylation of theobromine. In other words, it was confirmed that the DNAs of this group encode xanthosine methyltransferase, theobromine synthase and caffeine synthase, respectively. In addition, it was also found that the cellular extract of *Escherichia coli* catalyzed a productive reaction of 7-methylxanthine through ribose removal of 7-methylxanthosine. More specifically, it was found that the cellular extract of *Escherichia coli* which do not biologically synthesize methylxanthines in themselves also has an activity of an enzyme to remove the ribose moiety of 7-methylxanthosine (7-methylxanthosine hydrolase or 7-methylxanthosine phosphorolytic enzyme). Moreover, a mixture of the aforementioned three recombinant proteins and the cellular extract of *Escherichia coli* catalyzes a productive reaction from xanthosine to caffeine. In other words, it was confirmed that using the activities of the three methyltransferases and the activity of the one enzyme to remove the ribose moiety, a biosynthetic pathway of caffeine can be reconstituted in an *ex vivo,* i.e., cell free system, in other words, the pathway can be artificially constituted.

As the enzymes and cellular extract used in the reconstitution of the biosynthetic pathway of caffeine, those derived from any organism can be applied as long as they have comparable activities thereto. For example, instead of the theobromine synthase and caffeine synthase derived from coffee plants, methyltransferase derived from tea plants can be utilized, which catalyzes two steps of methylation reactions: 7-methylxanthine theobromine caffeine, as described in JP-A No. 2001-37490.

Moreover, reconstitution of the biosynthetic pathway of caffeine can be performed not only in a cell free system using the aforementioned enzymes and cellular extract, but also in a transformed biological system produced by introducing genes encoding these enzymes into any of organism species . Xanthosine which may become a starting material of biosynthesis of caffeine is present as an intermediate product of purine nucleotide catabolism in a wide range of organism species in addition to coffee plants and tea plants. Further, the enzyme to remove the ribose moiety of a purine nucleoside is known to react not only toward specific purine nucleoside derivatives but also toward various purine nucleoside derivatives, in general (see, Guranowski, Plant Physiol., 70, 344-349 (1982); Parkin, J. Biol. Chem., 271, 21713-21719 (1996); Ogawa et al., Appl. Environ. Microbiol., 67, 1783-1787 (2001)), and thus it is believed that the reaction of ribose removal of 7-methylxanthosine may be catalyzed by an enzyme to remove the ribose moiety of purine nucleoside, which is carried by general organisms. This can be readily expected also from the finding that *Escherichia coli* which do not biologically synthesize methylxanthines in themselves have an activity to remove the ribose moiety of 7-methylxanthosine (see, Examples herein). In brief, a biosynthetic pathway of caffeine can be reconstituted in wide variety of organism species through introducing and allowing the expression of a xanthosine methyltransferase gene, a theobromine synthase gene and a caffeine synthase gene.

The present invention was accomplished on the basis of the findings hereinabove.

### BRIEF DESCRIPTION OF THE DRAWINGS

Fig. 1 is a flow sheet illustrating a primary biosynthetic pathway of caffeine in coffee plants and tea plants. Name of each compound is described beneath each formula. SAM means S-adenosyl-L-methionine, and SAH means S-adenosyl-L-homocysteine. The reaction represented by (1) is catalyzed by xanthosine methyltransferase; the reaction represented by (2) is catalyzed by an enzyme to remove the ribose moiety of 7-methylxanthosine; the reaction represented by (3) is catalyzed by 7-methylxanthine methyltransferase (theobromine synthase); and the reaction represented by (4) is catalyzed by 3,7-dimethylxanthine methyltransferase (caffeine synthase).

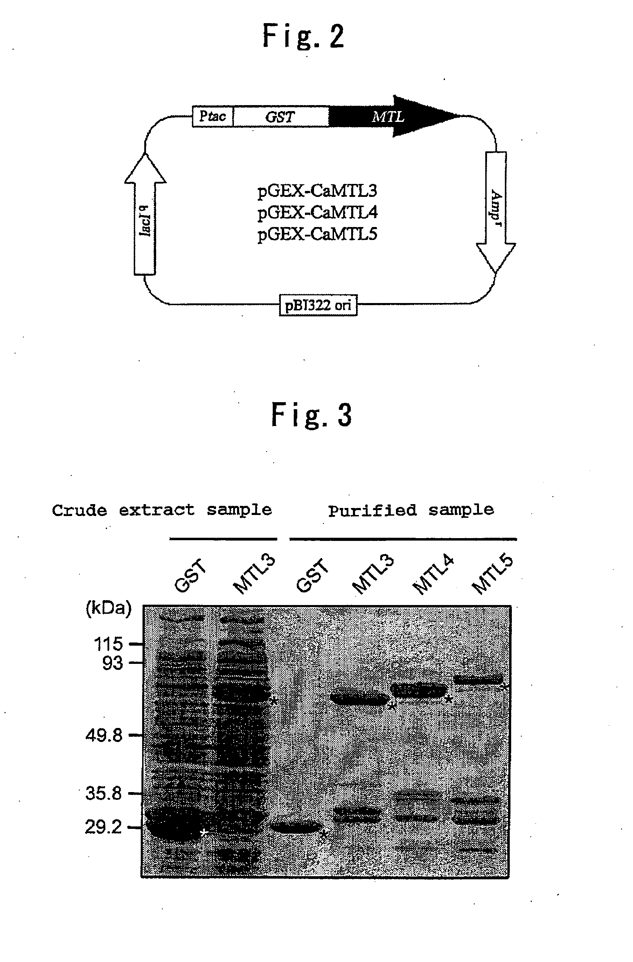

Fig. 2 is a drawing schematically illustrating pGEX-CaMTL3 vector, pGEX-CaMTL4 vector and pGEX-CaMTL5 vector. These vectors are those constructed by ligating CaMTL3 cDNA (SEQ ID NO: 2), CaMTL4 cDNA (SEQ ID NO: 5) or CaMTL5 cDNA (SEQ ID NO: 8), which is a predicted methyltransferase gene (MTL), downstream of a glutathione S-transferase gene (*GST*) controlled by tac promoter (P*tac*). Ampicillin resistant gene (*Amp*^{r}), lac repressor gene (*lacl*^{q}) and pBR322 replication origin (pBR322ori) are shown in the Figure.
Fig. 3 illustrates detection of recombinant proteins by the SDS-PAGE analysis. After separating crude extract sample of GST, crude extract sample of a fusion protein of GST and CaMTL3 (GST-MTL3, shown by crude extract sample MTL3 in Figure 3), purified sample of GST, purified sample of a fusion protein of GST and CaMTL3 (GST-MTL3, shown by purified sample MTL3 in Figure 3), purified sample of a fusion protein of GST and CaMTL4 (GST-MTL4, shown by purified sample MTL4 in Figure 3), and purified sample of a fusion protein of GST and CaMTL5 (GST-MTL5, shown by purified sample MTL5 in Figure 3) on a 9% gel, proteins were detected by Coomassie brilliant blue staining. Asterisks denote bands of the recombinant proteins.

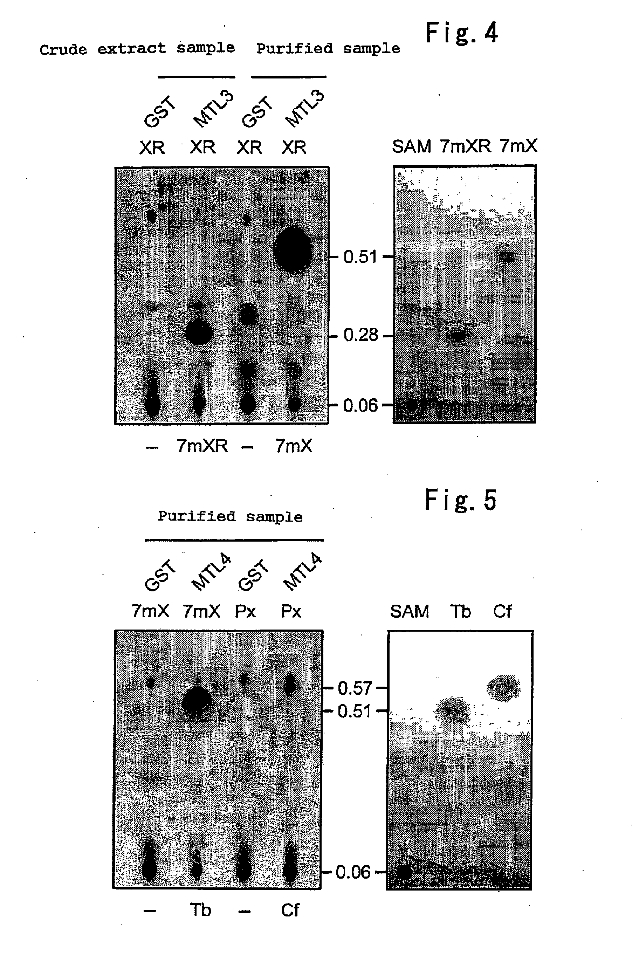

Fig. 4 illustrates detection by the thin layer chromatography (TLC) analysis of products of the enzymatic reactions. Enzymatic reactions were performed using the recombinant protein samples shown in Figure 3. Left panel illustrates states of detection by auto radiography of spots of products of the enzymatic reactions which were obtained by adding a methyl group (¹⁴C-labelled) after developing the enzymatic reaction mixture. Methyl group acceptors used in the enzymatic reactions are shown above the left panel, and specific products of the enzymatic reactions are shown beneath the left panel. Right panel illustrates states of detection by UV illumination of spots of standards after the standards were developed. Left and right panels were from one TLC plate which was divided in two parts after development. Starting points and the mobility are shown between the left and right panels. XR means xanthosine; 7mXR means 7-methylxanthosine; 7mX means 7-methylxanthine; and SAM means S-adenosyl-L-methionine.
Fig. 5 illustrates detection by the TLC analysis of products of the enzymatic reactions. Enzymatic reactions were performed using the recombinant protein samples shown in Figure 3. Left panel illustrates states of detection by auto radiography of spots of products of the enzymatic reactions which were obtained by adding a methyl group (¹⁴C-labelled) after developing the enzymatic reaction mixture. Methyl group acceptors used in the enzymatic reactions are shown above the left panel, and specific products of the enzymatic reactions are shown beneath the left panel. Right panel illustrates states of detection by UV illumination of spots of standards after the standards were developed. Left and right panels were from one TLC plate which was divided in two parts after development. Starting points and the mobility are shown between the left and right panels . 7mX means 7-methylxanthine; Tb means theobromine; Px means paraxanthine; Cf means caffeine; and SAM means S-adenosyl-L-methionine.

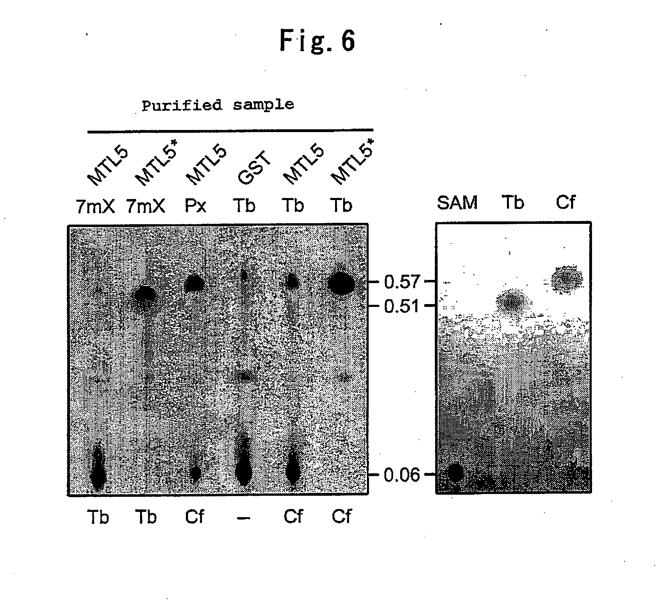

Fig. 6 illustrates detection by the TLC analysis of products of enzymatic reactions. Enzymatic reactions were performed using the recombinant protein samples shown in Figure 3. Left panel illustrates states of detection by auto radiography of spots of products of the enzymatic reactions which were obtained by adding a methyl group (¹⁴C-labelled) after developing the enzymatic reaction mixture or the purified product (lanes indicated by the asterisks). Methyl group acceptors used in the enzymatic reactions are shown above the left panel, and specific products of the enzymatic reactions are shown beneath the left panel. Right panel illustrates states of detection by UV illumination of spots of standards after the standards were developed. Left and right panels were from one TLC plate which was divided in two parts after development. Starting points and the mobility are shown between the left and right panels . 7mX means 7-methylxanthine; Tb means theobromine; Px means paraxanthine; Cf means caffeine; and SAM means S-adenosyl-L-methionine.

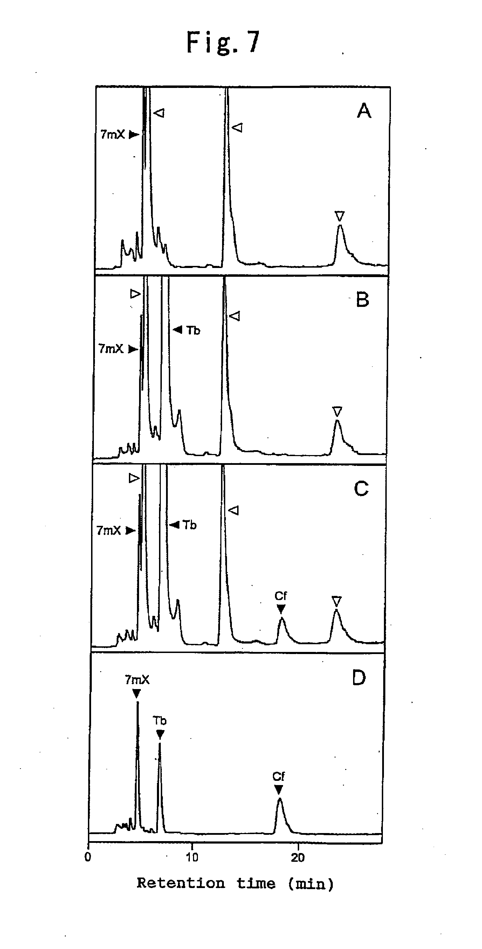

Fig. 7 illustrates detection of products of enzymatic reactions by an HPLC analysis. Enzymatic reactions were performed using the recombinant protein samples shown in Figure 3, andxanthosine alone as a methyl receptor. Chromatograms of the reaction mixtures are shown which were performed using: (A) crude extract MTL3 alone, (B) crude extract MTL3 and purified MTL4, and (C) crude extract MTL3, purified MTL4 and purified MTL5. (D) shows a chromatogram of the standards. Closed arrow heads indicate peaks of specific products and the standard. Open arrowheads indicate peaks of impurities. 7mX means 7-methylxanthine; Tb means theobromine; and Cf means caffeine.

### DETAILED DESCRIPTION OF THE INVENTION

The present invention is explained in detail below.

An aspect of the invention (first invention) is to provide a process for producing 7-methylxanthine, theobromine or caffeine which comprises: methylation of xanthosine at the 7-position of the purine ring; ribose removal of 7-methlxanthosine at the 9-position of the purine ring; methylation of 7-methylxanthine at the 3-position of the purine ring; and/or methylation of theobromine at the 1-position of the purine ring *ex vivo,* under the catalytic action of a combination of two or more of the following enzymes (a), (c) and (d), and the cellular extract (b):
(a) an enzyme having a catalytic activity of methylation of xanthosine at the 7-position of the purine ring and having the amino acid sequence set out in SEQ ID NO: 1,
(b) a crude cellular extract obtained from *Escherichia coli* having a catalytic activity of ribose removal of 7-methlxanthosine at the 9-position of the purine ring,
(c) an enzyme having a catalytic activity of methylation of 7-methylxanthine at the 3-position of the purine ring and having the amino acid sequence set out in SEQ ID NO: 4,
(d) an enzyme having a catalytic activity of methylation of theobromine at the 1-position of the purine ring and having the amino acid sequence set out in SEQ ID NO: 7.

In this first invention, at least one of the enzymes (a), (c) and (d), and the cellular extract (b) may be substituted for one having a comparable activity thereto. Next, second to eighth inventions relating to a process for reconstituting a biosynthetic pathway of caffeine in a transformed organism system according to the invention are explained.

Another aspect of the invention (second invention) relates to a process for producing theobromine or caffeine which comprises allowing the expression of a combination of two or more of the following DNA molecules (a), (b) and (c) in a host organism to alter the metabolism of the host organism. More particularly, the second invention is a process for producing theobromine or caffeine which comprises allowing the expression of a combination of (a) + (b) or a combination of (a) + (b) + (c) of the following DNA molecules (a), (b) and (c) in a host organism which biologically synthesizes xanthosine and has an enzymatic activity of ribose removal of 7-methylxanthosine to alter the metabolism of the host organism:
(a) a DNA molecule encoding an enzyme which catalyzes methylation of xanthosine at the 7-position of the purine ring and having the nucleotide sequence set out in SEQ ID NO: 2,
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

Yet another aspect of the invention (third invention) is to provide a process for producing theobromine or caffeine which comprises allowing the expression of a combination of the following DNA molecules (b) and (c) in a host organism which biologically synthesizes 7-methylxanthine to alter the metabolism of the host organism:
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

Still another aspect of the invention (fourth invention) is to provide a process for altering the amount of production of theobromine or caffeine which comprises allowing the expression of a combination of two or more of the following DNA molecules (a), (b) and (c) in a host organism which biologically synthesizes theobromine or caffeine:
(a) a DNA molecule encoding an enzyme which catalyzes methylation of xanthosine at the 7-position of the purine ring and having the nucleotide sequence set out in SEQ ID NO:2,
   a DNA molecule encoding an enzyme which catalyzes methylation of 7-methylxanthine at the 3-position of the purine ring and having the nucleotide sequence set out in SEQ ID NO: 5,
(b) a DNA molecule encoding an enzyme which catalyzes methylation of theobromine at the 1-position of the purine ring and having the nucleotide sequence set out in SEQ ID NO: 8.

In the second to fourth inventions, at least one of the DNA molecules (a), (b) and (c) maybe substituted for one having a comparable function thereto.

Further aspect of the invention (fifth invention) relates to a process for producing theobromine or caffeine which comprises allowing the expression of a combination of two or more of the following RNA molecules (a), (b) and (c) in a host organism to alter the metabolism of the host organism. More particularly, the fifth invention is a process for producing theobromine or caffeine which comprises allowing the expression of a combination of (a) + (b) or a combination of (a) + (b) + (c) of the following RNA molecules (a), (b) and (c) in a host organism which biologically synthesizes xanthosine and has an enzymatic activity of ribose removal of 7-methylxanthosine to alter the metabolism of the host organism:
(a) an RNA molecule encoding an enzyme which catalyzes methylation of xanthosine at the 7-position of the purine ring and having the nucleotide sequence set out in SEQ ID NO: 3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

Still another aspect of the invention (sixth invention) is to provide a process for producing theobromine or caffeine which comprises allowing the expression of a combination of the following RNA molecules (b) and (c) in a host organism which biologically synthesizes 7-methylxanthine to alter the metabolism of the host organism:
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

Yet another aspect of the invention (seventh invention) is to provide a process for altering the amount of production of theobromine or caffeine which comprises allowing the expression of a combination of two or more of the following RNA molecules (a), (b) and (c) in a host organism which biologically synthesizes theobromine or caffeine:
(a) an RNA molecule encoding an enzyme which catalyzes methylation of xanthosine at the 7-position of the purine ring and having the nucleotide sequence set out in SEQ ID NO:3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

In the fifth to seventh inventions, at least one of the RNA molecules (a), (b) and (c) may be substituted for one having a comparable function thereto.

Further aspect of the invention (eighth invention) is to provide a process according to any one of the second to seventh inventions wherein the host organism is a plant, and production or increase of production of theobromine or caffeine defends the host plant from pest feeding by a herbivore.

### DESCRIPTION OF THE PREFERRED EMBODIMENTS

The first invention relates to a process for reconstituting a biosynthetic pathway of caffeine in a cell free system, which is a process for producing 7-methylxanthine, theobromine or caffeine which comprises methylation of xanthosine at the 7-position of the purine ring, ribose removal of 7-methylxanthosine at the 9-position of the purine ring, methylation of 7-methylxanthine at the 3-position of the purine ring, and/or methylation of theobromine at the 1-position of the purine ring *ex vivo,* under the catalytic action of a combination of two or more of the following enzymes (a), (c) and (d), and the cellular extract (b):
(a) an enzyme having a catalytic activity of methylation of xanthosine at the 7-position of the purine ring and having the amino acid sequence set out in SEQ ID NO: 1,
(b) a crude cellular extract obtained from *Escherichia coli* having a catalytic activity of ribose removal of 7-methlxanthosine at the 9-position of the purine ring,
(c) an enzyme having a catalytic activity of methylation of 7-methylxanthine at the 3-position of the purine ring and having the amino acid sequence set out in SEQ ID NO: 4,
(d) an enzyme having a catalytic activity of methylation of theobromine at the 1-position of the purine ring and having the amino acid sequence set out in SEQ ID NO: 7.

Xanthosine methyltransferase which catalyzes methylation of xanthosine, theobromine synthase which catalyzes methylation of 7-methylxanthine, and caffeine synthase which catalyzes methylation of theobromine are generically referred to herein as caffeine synthetic methyltransferase.

In the first invention, at least one of the enzymes (a), (c) and (d), and the cellular extract (b) may be substituted for one having a comparable activity thereto. For example, instead of the theobromine synthase and caffeine synthase derived from coffee plants, methyltransferase derived from tea plants can be utilized, which catalyzes two steps of methylation reactions: 7-methylxanthine → theobromine → caffeine, as described in JP-A No. 2001-37490.

The aforementioned (a) and ones comparable thereto are generically referred to as xanthosine methyltransferase, the aforementioned (b) and ones comparable thereto are generically referred to as the enzyme to remove the ribose moiety of 7-methylxanthosine, the aforementioned (c) and ones comparable thereto are generically referred to as theobromine synthase, and the aforementioned (d) and ones comparable thereto are generically referred to as caffeine synthase herein, respectively.

Examples of the combination of the enzyme and cellular extract include a combination of (a) + (b) + (c) + (d) for synthesizing caffeine from xanthosine, a combination of (a) + (b) + (c) for synthesizing theobromine from xanthosine, and a combination of (c) + (d) for synthesizing caffeine from 7-methylxanthine.

The second invention which relates to a process for reconstituting a biosynthetic pathway of caffeine in a transformed organism system according to the invention is a process for producing theobromine or caffeine which comprises allowing the expression of a combination of (a) + (b) or a combination of (a) + (b) + (c) of the following DNA molecules (a), (b) and (c) in a host organism which biologically synthesizes xanthosine and has an enzymatic activity of ribose removal of 7-methylxanthosine to alter the metabolism of the host organism:
(a) a DNA molecule encoding an enzyme which catalyzes methylation of xanthosine at the 7-position of the purine ring and having the nucleotide sequence set out in SEQ ID NO: 2,
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

In the second invention, a combination of (a) + (b) + (c) is used for synthesizing caffeine from xanthosine, and a combination of (a) + (b) is used for synthesizing theobromine from xanthosine.

The third invention is a process for producing theobromine or caffeine which comprises allowing the expression of a combination of the following DNA molecules (b) and (c) in a host organism which biologically synthesizes 7-methylxanthine to alter the metabolism of the host organism:
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

The fourth invention is a process for altering the amount of production of theobromine or caffeine which comprises allowing the expression of a combination of two or more of the following DNA molecules (a), (b) and (c) in a host organism which biologically synthesizes theobromine or caffeine:
(a) a DNA molecule encoding an enzyme which catalyzes methylation of xanthosine at the 7-position of the purine ring and having the nucleotide sequence set out in SEQ ID NO:2,
(b) a DNA molecule encoding an enzyme which catalyzes methylation of 7-methylxanthine at the 3-position of the purine ring and having the nucleotide sequence set out in SEQ ID NO: 5.
(c) a DNA molecule encoding an enzyme which catalyzes methylation of theobromine at the 1-position of the purine ring and having the nucleotide sequence set out in SEQ ID NO: 8.

In the second to fourth inventions, at least one of the DNA molecules (a), (b) and (c) may be substituted for one having a comparable function thereto.

In the second to fourth inventions, 7-methylxanthine, theobromine or caffeine is obtained through methylation of xanthosine at the 7-positiion of the purine ring, ribose removal of 7-methylxanthosine at the 9-position of the purine ring, methylation of 7-methylxanthine at the 3-position of the purine ring, and/or methylation of theobromine at the 1-position of the purine ring.

The fifth invention is a process for producing theobromine or caffeine which comprises allowing the expression of a combination of (a) + (b) or a combination of (a) + (b) + (c) of the following RNA molecules (a), (b) and (c) in a host organism which biologically synthesizes xanthosine and has an enzymatic activity of ribose removal of 7-methylxanthosine to alter the metabolism of the host organism:
(a) an RNA molecule encoding an enzyme which catalyzes methylation of xanthosine at the 7-position of the purine ring and having the nucleotide sequence set out in SEQ ID NO: 3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

In the fifth invention, a combination of (a) + (b) + (c) is used for synthesizing caffeine from xanthosine, and a combination of (a) + (b) is used for synthesizing theobromine from xanthosine.

The sixth invention is a process for producing theobromine or caffeine which comprises allowing the expression of a combination of the following RNA molecules (b) and (c) in a host organism which biologically synthesizes 7-methylxanthine to alter the metabolism of the host organism:
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

The seventh invention is a process for altering the amount of production of theobromine or caffeine which comprises allowing the expression of a combination of two or more of the following RNA molecules (a), (b) and (c) in a host organism which biologically synthesizes theobromine or caffeine:
(a) an RNA molecule encoding an enzyme which catalyzes methylation of xanthosine at the 7-position of the purine ring and having the nucleotide sequence set out in SEQ ID NO:3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

In the fifth to seventh inventions, at least one of the RNA molecules (a), (b) and (c) may be substituted for one having a comparable function thereto.

In the fifth to seventh inventions, 7-methylxanthine, theobromine or caffeine is obtained through methylation of xanthosine at the 7-positiion of the purine ring, ribose removal of 7-methylxanthosine at the 9-position of the purine ring, methylation of 7-methylxanthine at the 3-position of the purine ring, and/or methylation of theobromine at the 1-position of the purine ring.

The eighth aspect of the invention is a process according to any one of second to seventh inventions wherein the host organism is a plant, and production or increase of production of theobromine or caffeine defends the host plant from pest feeding by a herbivore.

The DNA molecules encoding the aforementioned caffeine biosynthetic methyltransferases can be isolated from an organism which produces the caffeine biosynthetic methyltransferase utilizing for example, a PCR technique in which an origo base which specifically hybridizes thereto is used as a primer (PCR Experimental Protocol of Plants (Cell Technology, separate volume, Plant Cell Technology Series 2) Shujunsha Co. Ltd. (1995)).

Moreover, the DNA molecules encoding the aforementioned caffeine biosynthetic methyltransferases can be also isolated by performing hybridization screening of a cDNA library or a genomic library derived from an organism which produces the caffeine biosynthetic methyltransferase using the full length or partial DNA molecule thereof as a probe (Bio Experiments Illustrated 4, Cloning with Less Care (Cell Technology, separate volume, Visual Experimental Note Series) Shujunsha Co. Ltd. (1997)).

Such a DNA molecule encoding the caffeine biosynthetic methyltransferase obtained by the PCR technique or hybridization screening, or other method may have any nucleotide sequence as long as the translation product thereof has the intended methyltransferase activity, and may not necessarily has homology to any one of the sequence set out in SEQ ID NO: 2, 5 or 8.

Although the organism used for isolating the DNA molecules encoding the aforementioned caffeine biosynthetic methyltransferases may be any one as long as it produces methylxanthines, it is preferably a plant in genus coffee (Coffea), a plant in genus camellia (Camellia), a plant in genus cola (Cola), a plant in genus ilex (Ilex) and a plant in genus chocolate tree (Theobroma) for isolating a DNA molecule having the nucleotide sequence set out in SEQ ID NO: 2, 5 or 8, or a DNA molecule having homology to any one of these molecules.

The RNA molecules encoding the aforementioned caffeine biosynthetic methyltransferases can be obtained by ligating the caffeine biosynthetic methyltransferase DNA, which was obtained according to the aforementioned process, downstream of a promoter recognized by an RNA polymerase such as Sp6 RNA promoter or T7 promoter followed by transcription with Sp6 RNA polymerase, T7 RNA polymerase or the like. Alternatively, the RNA molecules can be also obtained through the use of the transcriptional activity of the host organism by forming a recombinant molecule through inserting a DNA or an RNA encoding the caffeine biosynthetic methyltransferase into an animal or plant virus or through inserting the caffeine biosynthetic methyltransferase DNA into an appropriate gene expression cassette as described hereinafter followed by introducing this recombinant molecule into the host organism.

For the expression and preparation of a recombinant protein from the DNA molecule or RNA molecule encoding the aforementioned caffeine biosynthetic methyltransferase, 1) a prokaryotic cellular expression system, 2) a yeast expression system, 3) a plant cellular expression system, 4) an insect cellular expression system, 5) a mammalian cellular expression system, 6) an *in vitro* transcription/translation system or the like can be utilized.

For obtaining the purified sample of the aforementioned recombinant protein, use of the prokaryotic cellular expression system (1) is most advantageous. For example, when GST Gene Fusion System (Amersham Biosciences) is used, the entire procedure starting from the construction of a vector to the expression and purification of the recombinant protein can be performed according to the attached instruction manual.

For identifying the function of the DNA molecule or RNA molecule encoding the aforementioned caffeine biosynthetic methyltransferase, a process is performed in which a recombinant protein (crude extract or purified product) is prepared by the aforementioned process, and an enzymatic reaction mixture containing the recombinant protein, a methyl group acceptor (xanthosine, 7-methylxanthine or theobromine) and a methyl group donor (S-adenosyl-L-methionine) is incubated followed by the TLC analysis or HPLC analysis of the product in this enzymatic reaction mixture (see, Examples herein).

Whether or not the biosynthetic pathway of caffeine can be reconstituted by way of the composite utilization of the DNA molecules or RNA molecules encoding the aforementioned caffeine biosynthetic methyltransferases can be determined by a procedure in which the recombinant proteins (crude extract or purified product) are prepared by the aforementioned process, and an enzymatic reaction mixture containing two or more recombinant proteins, a methyl group acceptor (xanthosine or 7-methylxanthine) and a methyl group donor (S-adenosyl-L-methionine) is incubated followed by the TLC analysis or HPLC analysis of the product in this enzymatic reaction mixture (see, Examples herein).

In the process for reconstituting the biosynthetic pathway of caffeine in a transformed organism system according to the invention, any organism species can be utilized as a host as long as it meets either requirement of: 1) biologically synthesizing xanthosine and having an enzymatic activity of ribose removal of 7-methylxanthosine; or 2) biologically synthesizing 7-methylxanthine.

Among the organisms which meet the above requirement, general plants are suitable as a host because they are comparably strong against toxicity of methylxanthines.

In connection with the process for reconstituting the biosynthetic pathway of caffeine in a transformed organism system according to the invention, examples in which a plant is used as a host is explained below.

For gene expression in a plant, any process can be utilized in which an expression cassette including 1) a promoter which enables transcription of from a DNA to an mRNA in host cells, 2) a caffeine biosynthetic methyltransferase DNA ligated downstream of the promoter in a sense direction, 3) a terminator sequence including a polyadenylation site required for stabilization of the transcription product ligated downstream of the DNA as needed, and the like is introduced into plant cells followed by transformation thereof.

The expression cassette may include a promoter for allowing the constitutive or inductive expression of the inserted DNA. Examples of the promoter for allowing the constitutive expression include 35S promoter of cauliflower mosaic virus, actin gene promoter of Oryza sativa, and the like. Examples of the promoter for allowing the inductive expression include chitinase gene promoter activated upon infection or invasion of a pathogenic microbe, PR protein gene promoter of tobacco, WIPK gene prompter of tobacco activated by lesion and the like.

Any of various techniques can be used for introducing the expression cassette into plant cells. Examples of these techniques include direct introduction methods (particle bombardment method, electroporation method and microinjection method), T-DNA introduction methods in which Agrobacterium tumefaciens is used as a transforming factor, and other possible techniques.

The direct introduction method does not require any specific vector. For example, simple plasmid such as a pUC derivative can be used. In the T-DNA introduction method, it is necessary to use a binary vector or the like containing a border sequence of T-DNA.

The transformed plant cell through introduction of the expression cassette can be converted into a plant tissue or a plant body via a regeneration process according to the conventional method.

For reconstituting the caffeine biosynthetic pathway, two or more types of expression cassettes must be introduced into plant cells.

When the direct introduction method is used, the transformant having a reconstituted biosynthetic pathway of caffeine can be obtained through the introduction of single vector containing multiple types of expression cassettes or multiple vectors containing single or multiple expression cassettes into plant cells. Alternatively, the transformant having a reconstituted biosynthetic pathway of caffeine can be also obtained through the introduction of single vector containing single or multiple expression cassettes into plant cells followed by mating of thus resulting transformant with other transformant.

When the T-DNA introduction method is used, the transformant having a reconstituted biosynthetic pathway of caffeine can be obtained through the introduction of single T-DNA containing multiple types of expression cassettes. Alternatively, the transformant having a reconstituted biosynthetic pathway of caffeine can be also obtained through the introduction of single T-DNA containing single or multiple expression cassettes into plant cells followed by mating of thus resulting transformant with other transformant.

A plant body (including tissue or cell) generated according to any of these methods or a plant body obtained from the propagation medium (such as seed, tuber or cut spike) has a reconstituted biosynthetic pathway of caffeine and produces at least one of the methylxanthines i.e., 7-methylxanthine, theobromine and caffeine.

The amount of production, the ratio of production and the like of methylxanthines in the plant having a reconstituted biosynthetic pathway of caffeine can be determined by the HPLC analysis. This HPLC analysis can be performed under a similar condition to those demonstrated in Examples herein.

Plants which produce theobromine or caffeine through the reconstitution of the biosynthetic pathway of caffeine have feeding-repellent effects and insecticidal effects against herbivore (insects, slug, snail and the like). In other words, such plant bodies endogenously have pesticidal effects.

### EXAMPLES

The present invention is specifically explained by way of the following examples . However, the scope of the invention is not limited to these examples.
(1) Synthesis of Double Stranded cDNA from mRNA Derived from Coffee Plants
   Total RNA was extracted from 5 g of immature fruits of coffee plants (Coffea arabica) by a CTAB method (see, Chang et al., Plant Mol. Biol. Rep., 11, 113-116 (1993)). Using PolyATtract mRNA Isolation System III (Promega), mRNA was purified from 100 µg of the total RNA. Double stranded cDNA was synthesized using 5 µg of the mRNA and ZAP-cDNA Synthesis Kit (Stratagene).
(2) Isolation of Novel cDNA having Homology to CaMXMT cDNA and CaMTL3 cDNA
   CaCS-N2 primer (5-ATGGAGCTCCAAGAAGTCCT-3) and CaCS-C1 primer (5-CTTTTACACGTCTGACTTCTCTG-3) were synthesized which were designed on the basis of a conserved sequence of CaMXMT cDNA (DDBJ/GenBank/EMBL accession number AB048794) and CaMTL3 cDNA (SEQ ID NO: 5, DDBJ/GenBank/EMBL accession number AB048793). PCR was carried out using the aforementioned cDNA, primers and Pyrobest DNA Polymerase (Takara Shuzo Co., Ltd.) to amplify a group of cDNA fragments. This group of cDNA fragments was inserted into EcoRV site of a vector pBluescript II KS-(Stratagene) to produce a plasmid library. Nucleotide sequence determination was conducted for plasmid clones randomly selected among the plasmid library, and CaMTL3 cDNA (SEQ ID NO: 2), and novel CaMTL4 cDNA (SEQ ID NO: 5) and CaMTL5 cDNA (SEQ ID NO: 8) having high homology thereto were isolated.
(3) Construction of GST Fusion Protein Expression Vector
   CaMTL3 cDNA, CaMTL4 cDNA and CaMTL5 cDNA were excised from the plasmid clones, and were inserted downstream of the glutathione S-transferase (GST) gene of pGEX-4T-2 vector (Amersham Biosciences), respectively. Through the procedure hereinabove, pGEX-CaMTL3 vector for allowing the expression of the fusion protein of GST and CaMTL3 (GST-MTL3), pGEX-CaMTL4 vector for allowing the expression of the fusion protein of GST and CaMTL4 (GST-MTL4), and pGEX-CaMTL5 vector for allowing the expression of the fusion protein of GST and CaMTL5 (GST-MTL5) were constructed. Thus resulting pGEX-CaMTL3 vector, pGEX-CaMTL4 vector and pGEX-CaMTL5 vector are schematically illustrated in Fig. 2. In Fig. 2, each vector was constructed by ligating CaMTL3 cDNA (SEQ ID NO: 2), CaMTL4 cDNA (SEQ ID NO: 5) or CaMTL5 cDNA (SEQ ID NO: 8), which is a predicted methyltransferase gene (MTL), downstream of a glutathione S-transferase gene (*GST*) controlled by tac promoter (P*tac*).
(4) Production of Recombinant Protein *Escherichia coli* BL21 were transformed with vector pGEX-4T-2, pGEX-CaMTL3, pGEX-CaMTL4 and pGEX-CaMTL5. Any of the transformants was subjected to shaking culture in 100 mL of 2 x YT liquid medium (supplemented with 100 mg/L ampicillin) at 37° C until the absorbance (A600) of the culture becomes about 0.7. To these cultures was added isopropyl-β-D-thiogalactoside (IPTG) to give the final concentration of 1 mM followed by additional shaking culture at 20° C for 16 hours. Through the procedure hereinabove, production of recombinant proteins (GST, GST-MTL3, GST-MTL4 and GST-MTL5) in *Escherichia coli* was executed.
(5) Crude Extraction and Purification of Recombinant Protein
   *Escherichia coli* including the produced recombinant protein were collected by centrifugation and suspended in 10 mL of a disintegrating wash fluid (50 mM Tris-HCl [pH 8.0], 1 mM EDTA, 5 mM dithiothreitol). The following procedure was carried out on ice or at 4°C. After thus resulting suspension was subjected to an ultrasonic disintegrating treatment, Triton X-100 was added thereto to give the final concentration of 1% followed by incubation for 30 minutes. Thus resulting disintegration fluid was separated to a supernatant and precipitates by centrifugation. This supernatant was referred to as a crude extract sample of the recombinant protein. To the crude extract sample was added a 100 µL glutathione Sepharose 4B resin (AmershamBiosciences), and the entire liquid was gently stirred for 30 minutes. The glutathione Sepharose 4B resin bound with the recombinant protein was recovered by centrifugation and washed three times with 1 mL of the disintegrating wash fluid. Thereafter, 100 µL of a lysis solution (50 mM Tris-HCl [pH 8.5], 1 mMEDTA, 5 mM dithiothreitol, 10 mM glutathione) was added to the resin, and the mixture was gently stirred for 15 minutes. From the mixture was recovered the eluate by centrifugation, which was referred to as a purified sample of the recombinant protein. SDS-PAGE analysis verified that the recombinant protein was included in the crude extract sample and the purified sample. Results of detection of the recombinant protein by the SDS-PAGE analysis are shown in Fig. 3.
(6) Identification of Enzymatic Reaction Product by Thin Layer Chromatography (TLC)
   Following enzymatic reaction experiments and TLC analyses were performed for the purified GST, crude extract GST, purified GST-MTL3, crude extract GST-MTL3, purified GST-MTL4 and purified GST-MTL5. An enzyme-containing mixture in an amount of 25 µL which comprises 100 mM Tris-HCl [pH 8.0], 200 µM MgCl₂, 500 µM substrate (xanthosine [XR], 7-methylxanthine [7mX] or theobromine [Tb]), 16.8 µMS-adenosyl-L-[methyl⁻¹⁴C]methionine [SAM] [2.2 GBq/mmol; Amersham Biosciences] and the recombinant protein sample (50 µg of the crude extract sample or 5 µg of the purified sample) was incubated at 27°C for 16 hours.
   The enzymatic reaction mixture after the incubation was filtered with a filter cup (ULTRAFREE-MC 10, 000 NMWL; Millipore) . The filtrate in an amount of 2 µL was mixed with 2 µL methanol, and the resulting mixture was spotted on a TLC plate (Silicagel 60 F254; Merck).
   Alternatively, the reaction product was purified by adding 250 µL of chloroform to the enzymatic reaction mixture after the incubation, vigorously stirring the entire solution, recovering the chloroform layer, which was subjected to concentration and evaporation to dryness. This purified product was suspended in 4 µL of 50% methanol, and spotted on a TLC plate.
   TLC analyses were performed similarly to those as described above using any one of 7-methylxanthosine [7mXR], [7mX], [Tb], caffeine [Cf] and S-adenosyl-L-methionine [SAM] (1 mM aqueous solution, each) as a standard, instead of the enzymatic reaction mixture and the purified product. A mixture of 4 µL of the standard and 4 µL of methanol was spotted on a TLC plate.
   Thereafter, development was conducted using water/acetic acid/n-butanol (2:1:4, v/v/v) as a developing solvent for 1.5 hrs. A sensitizing agent (En³ Hance; Dupont NEN) was sprayed onto the part with the developed reaction product present. Autoradiography was performed using an X-ray film (BioMax MS; Kodak) through the exposure at -80° C for 3 days to detect the spot of the enzymatic reaction product having a ¹⁴C-labelled methyl group added. The spot of the standard was detected by illuminating UV light onto the part with the developed standard. Results of analyses by TLC are illustrated in Fig. 4 to Fig. 6.
   As is clear from Fig. 4, the purified sample of GST-MTL3 produced [7mXR] in the presence of [XR] which is a methyl group acceptor and [SAM] which is a methyl group donor. In other words, it was revealed that this sample catalyzed a productive reaction of [7mXR] through methylation of [XR].
   Further, the crude extract sample of GST-MTL3 produced [7mX] which is a ribose moiety-removed product of [7mXR] in the presence of [XR] which is a methyl group acceptor and [SAM] which is a methyl group donor. In other words, it was revealed that this sample catalyzed two reactions of producing [7mXR] through methylation of [XR] and producing [7mX] through ribose removal of [7mXR].
   As is clear from Fig. 5, the purified sample of GST-MTL4 produced [Tb] in the presence of [7mX] which is a methyl group acceptor and [SAM] which is a methyl group donor. In other words, it was revealed that this sample catalyzed a productive reaction of [Tb] through methylation of [7mX].
   Further, the purified sample of GST-MTL4 produced [Cf] in the presence of paraxanthine [Px] which is a methyl group acceptor and [SAM] which is a methyl group donor. In other words, it was revealed that this sample catalyzed a productive reaction of [Cf] through methylation of [Px].
   As is clear from Fig. 6, the purified sample of GST-MTL5 produced [Cf] in the presence of [Tb] which is a methyl group acceptor and [SAM] which is a methyl group donor. In other words, it was revealed that this sample catalyzed a productive reaction of [Cf] through methylation of [Tb].
   Further, the purified sample of GST-MTL5 produced [Tb] in the presence of [7mX] which is a methyl group acceptor and [SAM] which is a methyl group donor. In other words, it was revealed that this sample catalyzed a productive reaction of [Tb] through methylation of [7mX].
   Moreover, the purified sample of GST-MTL5 produced [Cf] in the presence of [Px] which is a methyl group acceptor and [SAM] which is a methyl group donor. In other words, it was revealed that this sample catalyzed a productive reaction of [Cf] through methylation of [Px].
   GST which was used as a control did not catalyze any methylation reaction in both of the purified sample and the crude extract sample.
   Accordingly, it was revealed that the CaMTL3 protein, CaMTL4 protein and CaMTL5 protein, and a group of genes encoding these proteins are constituent elements of the biosynthetic pathway of caffeine, respectively.
(7) Reconstitution of Biosynthetic Pathway of Caffeine *in vitro*
   An enzyme-containing mixture in an amount of 100 µL which comprises 100mMTris-HCl [pH8.0], 200 µM MgCl₂, 500 µM xanthosine [XR], 1.5 mM S-adenosyl-L-methionine [SAM] and the recombinant protein sample (200 µg of the crude extract sample or 20 µg of the purified sample) was incubated at 27°C for 16 hours. The aforementioned recombinant protein samples used in the enzymatic reaction were: (A) crude extract GST-MTL3 alone, (B) combination of crude extract GST-MTL3 and purified GST-MTL4, and (C) combination of crude extract GST-MTL3, purified GST-MTL4 and purified GST-MTL5.

The reaction product was purified by adding 1 mL of chloroform to the enzymatic reaction mixture after the incubation, vigorously stirring the entire solution, recovering the chloroform layer, which was subjected to concentration and evaporation to dryness. This purified product was suspended in 200 µL of an HPLC developing solvent (50 mM sodium phosphate [pH 6.0]/methanol [4:1, v/v]) and 20 µL of the suspension was used for the HPLC analysis. Puresil C18 (Waters) was used as a column for the HPLC analysis, and the suspension was developed at a flow rate of 1 mL/min. The enzymatic reaction product was detected by absorption of UV light (270 nm). HPLC analyses were performed similarly to those as described above using any one of 7-methylxanthine [7mX], theobromine [Tb] and caffeine [Cf] as a standard, instead of the enzymatic reaction product. Results of analyses by HPLC are illustrated in Fig. 7.

As is clear from Fig. 7-A, the crude extract sample of GST-MTL3 produced [7mX] which is a ribosemoiety-removed product of [7mXR] in the presence of [XR] which is a methyl group acceptor and [SAM] which is a methyl group donor. In other words, it was determined that this sample catalyzed two reactions of producing [7mXR] through methylation of [XR] and producing [7mX] through ribose removal of [7mXR] in the HPLC analysis as well similarly to the TLC analysis.

As is clear from Fig. 7-B, a combination of the crude extract sample of GST-MTL3 and the purified sample of GST-MTL4 produced [7mX] and [Tb] in the presence of [XR] and [SAM]. In other words, it was revealed that the combination of these samples catalyzed three reactions of producing [7mXR] through methylation of [XR], producing [7mX] through ribose removal of [7mXR], and producing [Tb] through methylation of [7mX].

As is clear from Fig. 7-C, a combination of the crude extract sample of GST-MTL3, the purified sample of GST-MTL4 and the purified sample of GST-MTL5 produced [7mX], [Tb] and [Cf] in the presence of [XR] and [SAM]. In other words, it was revealed that the combination of these samples catalyzed four reactions of producing [7mXR] through methylation of [XR], producing [7mX] through ribose removal of [7mXR], producing [Tb] through methylation of [7mX], and producing [Cf] through methylation of [Tb].

As in the foregoing, it was proven that a biosynthetic pathway of caffeine can be reconstituted *in vitro* through composite utilization of the CaMTL3 protein, CaMTL4 protein, CaMTL5 protein and crude extract of *Escherichia coli.* In accordance with the present invention, effects as described below are exerted.
(1) Using two or more of the multiple enzymes in combination which respectively catalyze each step in a series of the caffeine biosynthetic reaction system, 7-methylxanthine, theobromine or caffeine can be produced *ex vivo* through executing methylation of xanthosine at the 7-position of the purine ring, ribose removal of 7-methylxanthosine at the 9-position of the purine ring, methylation of 7-methylxanthine at the 3-position of the purine ring, and/or methylation of theobromine at the 1-position of the purine ring.
(2) Using two or more of the multiple genes in the biosynthetic pathway of caffeine in combination which encode multiple enzymes which respectively catalyze each step in a series of the biosynthetic reaction system of caffeine, the amount of production of theobromine or caffeine can be altered through allowing the expression of this combination in a host organism which biologically synthesizes theobromine or caffeine.
(3) Using two or more of the multiple genes in the biosynthetic pathway of caffeine in combination which encode multiple enzymes which respectively catalyze each step in a series of the biosynthetic reaction system of caffeine, theobromine or caffeine can be produced through allowing the expression of this combination in a host organism which biologically synthesizes 7-methylxanthine to alter the metabolism of the host organism.
(4) Using two or more of the multiple genes in the biosynthetic pathway of caffeine in combination which encode multiple enzymes which respectively catalyze each step in a series of the biosynthetic reaction system of caffeine, theobromine or caffeine can be produced through allowing the expression of the combination in a host organism which biologically synthesizes xanthosine and has an enzymatic activity of ribose removal of 7-methylxanthosine to alter the metabolism of the host organism.
(5) According to the process of the above (3) or (4), metabolism of the organism which does not biologically synthesize theobromine or caffeine in itself is altered to allow the organism to produce these compounds.
(6) Through performing the aforementioned process (3) or (4) in a plant, it becomes possible to defend the plant from pest feeding by a herbivore.

## Claims

1. A process for producing 7-methylxanthine, theobromine or caffeine which comprises: methylation of xanthosine at the 7-position of the purine ring; ribose removal of 7-methylxanthosine at the 9-position of the purine ring; methylation of 7-methylxanthine at the 3-position of the purine ring; and/or methylation of theobromine at the 1-position of the purine ring *ex vivo,* under the catalytic action of a combination of two or more of the following enzymes (a), (c) and (d), and the cellular extract (b):
(a) an enzyme having a catalytic activity of methylation of xanthosine at the 7-position of the purine ring and having the amino acid sequence set out in SEQ ID NO: 1,
(b) a crude cellular extract obtained from *Escherichia coli* having a catalytic activity of ribose removal of 7-methylxanthosine at the 9-position of the purine ring,
(c) an enzyme having a catalytic activity of methylation of 7-methylxanthine at the 3-position of the purine ring and having the amino acid sequence set out in SEQ ID NO: 4,
(d) an enzyme having a catalytic activity of methylation of theobromine at the 1-position of the purine ring and having the amino acid sequence set out in SEQ ID NO: 7.

2. The process according to claim 1 wherein at least one of the enzymes (a), (c) and (d), and the cellular extract (b) is substituted for one having a comparable activity thereto.

3. A process for producing theobromine or caffeine which comprises allowing the expression of a combination of (a) + (b) or a combination of (a) + (b) + (c) of the following DNA molecules (a), (b) and (c) in a host organism which biologically synthesizes xanthosine and has an enzymatic activity of ribose removal of 7-methylxanthosine to alter the metabolism of the host organism:
(a) a DNA molecule encoding an enzyme which catalyzes methylation of xanthosine at the 7-position of the purine ring and having the nucleotide sequence set out in SEQ ID NO: 2,
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

4. A process for producing theobromine or caffeine which comprises allowing the expression of a combination of the following DNA molecules (b) and (c) in a host organism which biologically synthesizes 7-methylxanthine to alter the metabolism of the host organism:
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

5. A process for altering the amount of production of theobromine or caffeine which comprises allowing the expression of a combination of two or more of the following DNA molecules (a), (b) and (c) in a host organism which biologically synthesizes theobromine or caffeine:
(a) a DNA molecule encoding an enzyme which catalyzes methylation of xanthosine at the 7-position of the purine ring and having the nucleotide sequence set out in SEQ ID NO:2,
(b) a DNA molecule encoding an enzyme which catalyzes methylation of 7-methylxanthine at the 3-position of the purine ring and having the nucleotide sequence set out in SEQ ID NO: 5,
(c) a DNA molecule encoding an enzyme which catalyzes methylation of theobromine at the 1-position of the purine ring and having the nucleotide sequence set out in SEQ ID NO: 8.

6. The process according to any one of claims 3 to 5 wherein at least one of the DNA molecules (a), (b) and (c) is substituted for one having a comparable function thereto.

7. A process for producing theobromine or caffeine which comprises allowing the expression of a combination of (a) + (b) or a combination of (a) + (b) + (c) of the following RNA molecules (a), (b) and (c) in a host organism which biologically synthesizes xanthosine and has an enzymatic activity of ribose removal of 7-methylxanthosine to alter the metabolism of the host organism:
(a) an RNA molecule encoding an enzyme which catalyzes methylation of xanthosine at the 7-position of the purine ring and having the nucleotide sequence set out in SEQ ID NO: 3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

8. A process for producing theobromine or caffeine which comprises allowing the expression of a combination of the following RNA molecules (b) and (c) in a host organism which biologically synthesizes 7-methylxanthine to alter the metabolism of the host organism:
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

9. A process for altering the amount of production of theobromine or caffeine which comprises allowing the expression of a combination of two or more of the following RNA molecules (a), (b) and (c) in a host organism which biologically synthesizes theobromine or caffeine:
(a) an RNA molecule encoding an enzyme which catalyzes methylation of xanthosine at the 7-position of the purine ring and having the nucleotide sequence set out in SEQ ID NO:3,
(b) an RNA molecule encoding an enzyme which catalyzes methylation of 7-methylxanthine at the 3-position of the purine ring and having the nucleotide sequence set out in SEQ ID NO: 6,
(c) an RNA molecule encoding an enzyme which catalyzes methylation of theobromine at the 1-position of the purine ring and having the nucleotide sequence set out in SEQ ID NO: 9.

10. The process according to any one of claims 7 to 9 wherein at least one of the RNA molecules (a), (b) and (c) is substituted for one having a comparable function thereto.

11. The process according to any one of claims 3 to 10 wherein the host organism is a plant, and production or increase of production of theobromine or caffeine defends the host plant from pest feeding by a herbivore.
